# EUROPÄISCHE PATENTSCHRIFT

(11) **EP 0 809 628 B1**
(45) Veröffentlichungstag und Bekanntmachung des Hinweises auf die Patenterteilung: **21.02.2001**
(21) Anmeldenummer: 95940197.7
(22) Anmeldetag: 17.11.1995
(51) Int. Cl.: C07C 309/17, C07C 309/19, C07C 309/22, A61K 7/00, C11D 1/66, B01F 17/00

(54) **AMPHIPHILE VERBINDUNGEN MIT MINDESTENS ZWEI HYDROPHILEN UND MINDESTENS ZWEI HYDROPHOBEN GRUPPEN AUF DER BASIS VON DICARBONSÄUREDIESTERN**
AMPHIPHILIC COMPOUNDS WITH AT LEAST TWO HYDROPHILIC AND AT LEAST TWO HYDROPHOBIC GROUPS BASED ON DICARBOXYLIC ACID DIAMIDES
COMPOSES AMPHIPHILES, COMPORTANT AU MOINS DEUX GROUPEMENTS HYDROPHILES ET AU MOINS DEUX GROUPEMENTS HYDROPHOBES, A BASE DE DIESTERS D'ACIDES DICARBOXYLIQUES

(30) Priorität: 17.02.1995 DE 19505367
(43) Veröffentlichungstag der Anmeldung: 03.12.1997
(73) Patentinhaber: RWE-DEA Aktiengesellschaft für Mineraloel und Chemie, 22297 Hamburg (DE)
(72) Erfinder: KWETKAT, Klaus, D-44534 Lünen (DE); RUBACK, Wulf, D-48249 Dülmen (DE)
(74) Vertreter: Schupfner, Gerhard D., Dr. Dipl.-Chem.
(86) Internationale Anmeldenummer: EP9504531
(87) Internationale Veröffentlichungsnummer: WO9625393

(56) Entgegenhaltungen:
- DE-A- 3 932 492
- PATENT ABSTRACTS OF JAPAN vol. 950 no. 001 & JP,A,07 003287 (SAKATANI TAKENOBU) 6.Januar 1995,
- CHEMICAL ABSTRACTS, vol. 107, no. 22, 30.November 1987 Columbus, Ohio, US; abstract no. 200971, KUDRYASHOVA ET AL. 'Atomic absorption determination of sodium and silicon in organosilicon and organosilicon surfactants' & ZH. ANAL. KHIM., Bd. 42, Nr. 6, 1987 Seiten 1036-1040,
- CHEMICAL ABSTRACTS, vol. 98, no. 12, 21.März 1983 Columbus, Ohio, US; abstract no. 98705, KASHLINSKAYA ET AL. 'Study of the interaction of gelatin and surface-active agents by turbidimetry' & KOLLOIDN. ZH., Bd. 44, Nr. 6, 1982 Seiten 1170-1173,
- PATENT ABSTRACTS OF JAPAN vol. 016 no. 383 (C-0974) ,17.August 1992 & JP,A,04 124165 (KANEBO LTD) 24.April 1994, in der Anmeldung erwähnt
- PATENT ABSTRACTS OF JAPAN vol. 014 no. 090 (C-0691) ,20.Februar 1990 & JP,A,01 304033 (LION CORP.) 7.Dezember 1989, in der Anmeldung erwähnt

## Beschreibung

Die Erfindung betrifft amphiphile Verbindungen mit mindestens zwei hydrophilen und mindestens zwei hydrophoben Gruppen auf der Basis von Dicarbonsäurediestern.

Als amphiphile Substanzen sind eine große Vielfalt an anionischen, kationischen, nichtionischen und zwitterionischen Verbindungen bekannt. Die weitaus meisten dieser Substanzen bestehen aus einer hydrophilen Kopfgruppe und wenigstens einem hydrophoben Teil.

Bei den amphiphilen Substanzen gibt es aus ökologischen Gründen, z. B. bezüglich der Verringerung des Verpackungs- und Transportaufwandes, die Notwendigkeit, immer größere Wirkung pro Masse an eingesetzter Substanz zu erzielen. Da eine Optimierung durch Mischung von amphiphilen Substanzen nur sehr begrenzt weiterführt, sind neue amphiphile Substanzen mit einem höheren Wirkungsgrad erforderlich. Es müssen daher insbesondere Stoffe mit niedrigeren kritischen Micellbildungskonzentrationen und/oder niedrigeren Ober- und Grenzflächenspannungen gefunden werden, um die Einsatzmengen an Wirksubstanz deutlich reduzieren zu können.

Erste Lösungsansätze in dieser Richtung durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G. Beinert, P. Marie, R. Zana, Langmuir, 9 (1993) 1465].

Anionische grenzflächenaktive Verbindungen mit wenigstens zwei hydrophilen und wenigstens zwei hydrophoben Gruppen sind bisher nur auf der Basis von Diglycidylethern hergestellt worden (US 5 160 450, JP 01 304 033, JP 4 124 165). Diglycidylether gelten jedoch als toxikologisch bedenklich und sind recht teuer. Darüber hinaus wird für ihre Herstellung Epichlorhydrin verwendet, was zu großen Mengen an Reststoffen führt, so daß diese Verbindungen unter ökotoxikologischen wie auch ökonomischen Gesichtspunkten nicht mehr zeitgemäß sind.

Es bestand daher die Aufgabe, amphiphile Verbindungen aufzufinden, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne großen Anfall von unerwünschten Nebenprodukten hergestellt werden können.

Die Aufgabe wird erfindungsgemäß durch sulfonierte, amphiphile Dicarbonsäurediester, die z. B. durch Sulfonierung von Dicarbonsäuredifettalkoholestern oder durch Sulfonierung von Dicarbonsäuredialkylestern mit kurzen Alkylketten und anschließende Umesterung mit Fettalkoholen und Neutralisation der Sulfonsäuren hergestellt werden können, gelöst.

Bei den erfindungsgemäßen amphiphilen Verbindungen handelt es sich um Verbindungen der allgemeinen Formel I 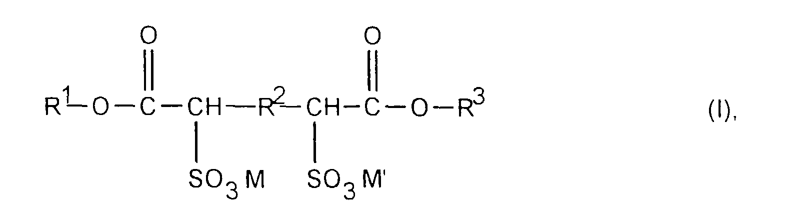 wobei
R¹, R² und R³ in der Formel I die im folgenden beschriebenen Bedeutungen haben:
R¹ und R³ stehen unabhängig voneinander für einen unverzweigten oder verzweigten gesättigten oder ungesättigten Kohlenwasserstoffrest mit 1 bis 22, vorzugsweise 8 bis 18, Kohlenstoffatomen.

Es seien als Substituenten R¹ und R³ im einzelnen die Reste Methyl, Ethyl. n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Henicosyl, n-Docosyl und ihre verzweigtkettigen Isomeren sowie die entsprechenden einfach, zweifach oder dreifach ungesättigten Reste genannt.

Der Spacer R² bedeutet im einzelnen
◆ unverzweigte oder verzweigte Alkylenketten der Formel II

   -CₐH₂ₐ- (II)

   mit a = 2 bis 18, vorzugsweise a = 2 bis 6;
◆ unverzweigte oder verzweigte Alkinylenketten der Formel III

   -C_{d}H_{2d}-C≡C-CₑH₂ₑ- (III)

   mit d + e = 2 bis 16, wobei d und e jeweils größer als Null ist,
◆ Alicyclen gemäß der Formel IV

   -C_{f}H_{2f}-cyclo C₆H₁₀-C_{g}H_{2g}- (IV)

   mit f und g gleich unabhängig voneinander je 1 bis 6 oder gemäß der Formel V

   -3(4),8(9)-di(methylen)-tricyclo[5.2.1.0^{2.6}]decan- (V);
◆ unsubstituierte oder substituierte Aromaten gemäß der Formel VI

   -CₕH₂ₕ-C₆R₄-(CᵢH₂ᵢ-C₆R₄)_{j₁}-C_{j₂}H_{2j₂}- (VI)

   oder gemäß der Formel VII

   -CₕH₂ₕ-C₁₀R₆-CⱼH₂ⱼ- (VII)

   mit h, j, j₁ und j₂ gleich unabhängig voneinander je 0 bis 8 und i = 0 bis 8 und
   mit R gleich unabhängig voneinander jeweils H oder C₁- bis C₆-Alkyl;
◆ eine Gruppe gemäß der Formel VIII

   -CₖH₂ₖ-CₓR_{y}-Z-CₓR_{y}-C_{I}H_{2I}- (VIII)

   mit k und l gleich unabhängig voneinander je 0 bis 8, x = 6 und y = 4 oder x = 10 und y = 6 oder x = 14 und y = 8, und
   Z = O, CO, NH, N-CH₂-CH(OX)-R¹, NR¹, N-C(O)R¹, SO₂
   oder gemäß der Formel IX

   -CH₂-CH(OCH₂CH(OX)-R¹)-CH₂- oder ein Isomer (IX)

   mit X = SO₃M
   oder 2,2'-Methylen-bis-(1,3-dioxolan-5-methylen)-oder Acetale, insbesondere Diacetale aus Dialdehyden und Di-, Oligo- oder Polyolen,
   wobei R unabhängig voneinander H oder C₁-C₆-Alkyl und R¹ einen Kohlenwasserstoffrest mit 1 bis 22 Kohlenstoffatomen bedeutet,
◆ eine Gruppe gemäß der Formel X

   -CₘH₂ₘ-(CₙH₂ₙO)ₚ(C₃H₆O)_{q}-CᵣH₂ᵣ- (X)

   mit m = 1 bis 4, n = 2 bis 4, p = 1 bis 20, vorzugsweise p = 1 bis 4, q = 0 bis 4 und r = 1 bis 4,
   wobei auch gemischte Alkoxideinheiten auftreten können und dann die Reihenfolge der Alkoxideinheiten beliebig ist,
◆ eine Gruppe gemäß der Formel XI

   -CᵣH₂ᵣ(RNCₛH₂ₛ)ₜ-CᵤH₂ᵤ- (XI)

   oder gemäß der Formel XII

   -[CᵣH₂ᵣ[RN-C(O)-NR]ₜ-CᵤH₂ᵤ]_{w}- (XII)

   oder gemäß der Formel XIII

   -[CᵣH₂ᵣ[RNC(O)CᵥH₂ᵥC(O)NR]ₜ-CᵤH₂ᵤ]_{w}- (XIII)

   oder gemäß der Formel XIV

   -[CᵣH₂ᵣ[RNC(O)CₓR_{y}C(O)NR]ₜ-CᵤH₂ᵤ]_{w}- (XIV)

   mit r = 2 bis 4, s = 2 bis 4, t = 1 bis 20, vorzugsweise t = 1 bis 4, u = 2 bis 4,
   v = 0 bis 12, w = 1 bis 6, x = 6 und y = 4 oder
   x = 10 und y = 6 oder
   x = 14 und y = 8
   mit R gleich unabhängig voneinander H oder C₁- bis C₆-Alkyl,
   mit M, M' = Alkali, Ammonium-, Alkanolammonium oder ½ Erdalkali.

In der deutschen Offenlegungsschrift DE 39 32 492 werden Salze von sulfonierten Estern ungesättigter Dicarbonsäuren mit ungesättigten Fettalkoholen beschrieben. Diese Verbindungen weisen jedoch Strukturen auf, die von denen der erfindungsgemäßen Verbindungen völlig verschieden sind, da in DE 39 32 492 SO₃ ausschließlich an Doppelbindungen addiert wird.

Die erfindungsgemäßen amphiphilen Verbindungen zeichnen sich meist durch extrem niedrige kritische Micellbildungskonzentrationen (CMC) und sehr niedrige Oberflächen- und Grenzflächenspannungen (z. B. gegen Paraffin) aus, was auf ihre besondere Struktur - wenigstens zwei hydrophile Gruppen und wenigstens zwei hydrophobe Gruppen - zurückgeführt werden muß. Darüber hinaus weisen die meisten von ihnen ein recht hohes hydrophiles Suspendiervermögen auf, das etwa auf halbem Wege zwischen dem konventioneller Tenside und dem des Pentanatriumtripolyphosphats liegt. Einige dieser Verbindungen sind extrem schnelle Netzmittel.

Die amphiphilen Verbindungen gemäß dieser Erfindung eignen sich insbesondere als Emulgatoren, Demulgatoren, Detergenzien, Dispergatoren und Hydrotropica in Industrie und Haushalt, beispielsweise auf den Gebieten Metallbearbeitung, Erzgewinnung, Oberflächenveredelung, Waschen und Reinigen, Kosmetik, Medizin und Nahrungsmittelverarbeitung und -zubereitung.

Hierbei können sie mit allen gängigen anionischen, nichtionischen, kationischen und ampholytischen grenzflächenaktiven Substanzen kombiniert werden. Als Beispiele für nichtionische grenzflächenaktive Substanzen, die für eine Kombination eingesetzt werden können, seien Fettsäureglyceride, Fettsäurepolyglyceride, Fettsäureester, Ethoxylate höherer Alkohole, Polyoxyethylenfettsäureglyceride, Polyoxyethylenpropylenglykolfettsäureester, Polyoxyethylensorbitanfettsäureester, Polyoxyethylen-Rhizinusöl- oder gehärtete Rhizinusöl-Derivate, Polyoxyethylenlanolinderivate, Polyoxyethylenfettsäureamide, Polyoxyethylenalkylamine, Alkanolamine, Alkyl-aminoxide, Derivate von Eiweißhydrolysaten, Hydroxymischether, Alkylpolyglycoside und Alkylglucamide genannt.

Als Beispiele für anionische grenzflächenaktive Substanzen, die für Kombinationen eingesetzt werden können, seien Seifen, Ethercarbonsäuren und deren Salze, Alkylsulfonate, α-Olefinsulfonate, Sulfonate höherer Fettsäureester, Alkoholsulfate, Alkoholethersulfate, Hydroxymischethersulfate, Salze von Phosphatestern, Tauride, Isethionate, lineare Alkylbenzolsulfonate, Cumolsulfonat, Alkylarylsulfonate, Sulfate der Polyoxyethylenfettsäureamide und Salze von Acylaminosäuren genannt.

Als Beispiele für kationische gängige grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Alkylpyridiniumsalze, Alkylisochinoliniumsalze, Benzethoniumchloride und kationische Acylaminosäurederivate genannt.

Als Beispiele für ampholytische grenzflächenaktive Substanzen, die für Kombinationen eingesetzt werden können, seien Aminosäuren, Betaine, Sulfobetaine, Imidazolinderivate, Sojaöllipide und Lecithin genannt.

Darüber hinaus können die erfindungsgemäßen amphiphilen Verbindungen auch für sich miteinander kombiniert werden.

Den erfindungsgemäßen amphiphilen Verbindungen können ebenfalls gängige Additive zugesetzt werden. Solche Additive werden speziell für eine Formulierung ausgewählt und umfassen üblicherweise anorganische Salze, wie Natriumchlorid und -sulfat, sowie Builder, Hydrotropica, UV-Absorber, Weichmacher, Chelatbildner, Viskositätsmodifizierer und Riechstoffe.

Die obengenannten Verbindungen lassen sich z. B. durch doppelte Sulfonierung der Dicarbonsäuredifettalkoholester oder durch doppelte Sulfonierung von Dicarbonsäuredialkylestern mit kurzen Alkylketten und anschließende Umesterung mit Fettalkoholen und Neutralisation der Sulfonsäuren mit wäßrigen Alkali- oder Erdalkalihydroxiden oder Ammoniak oder Alkanolaminen herstellen. Bei Bedarf werden die Produkte in wäßriger Lösung mit Wasserstoffperoxid (0,1 bis 2,0 %, bezogen auf Feststoff) gebleicht.

## Patentansprüche

1. Amphiphile Verbindungen der allgemeinen Formel I 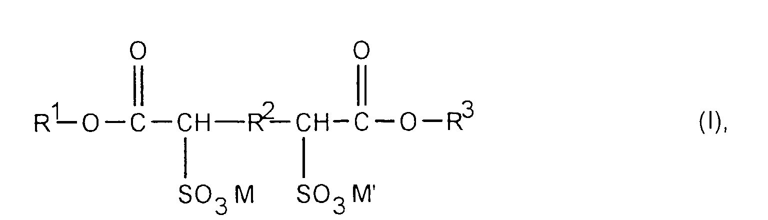
in der R¹ und R³ unabhängig voneinander einen unverzweigten oder verzweigten, gesättigten oder ungesättigten Kohlenwasserstoffrest mit 1 bis 22 Kohlenstoffatomen,
R²
◆ eine unverzweigte oder verzweigte Alkylenkette der Formel II
-CₐH₂ₐ- (II)
mit a = 2 bis 18,
◆ eine unverzweigte oder verzweigte Alkinylenkette der Formel III
-C_{d}H_{2d}-C≡C-CₑH₂ₑ- (III)
mit d + e = 2 bis 16, wobei d und e jeweils größer als Null sind,
◆ Alicyclen gemäß der Formel IV
-C_{f}H_{2f}-cyclo C₆H₁₀-C_{g}H_{2g}- (IV)
mit f und g gleich unabhängig voneinander je 1 bis 6 oder gemäß der Formel V
-3(4),8(9)-di(methylen)-tricyclo[5.2.1.0^{2.6}]decan- (V)
◆ unsubstituierte oder substituierte Aromaten gemäß der Formel VI
-CₕH₂ₕ-C₆R₄-CᵢH₂ᵢC₆R₄)_{j₁}-C_{j₂}H_{2j₂}- (VI)
oder gemäß der Formel VII
-CₕH₂ₕ-C₁₀R₆-CⱼH₂ⱼ- (VII)
mit h, j, j₁ und j₂ gleich unabhängig voneinander je 0 bis 8 und i = 0 bis 8 und
mit R gleich unabhängig voneinander jeweils H oder C₁- bis C₆-Alkyl
◆ eine Gruppe gemäß der Formel (VIII)
-CₖH₂ₖ-CₓR_{y}-Z-CₓR_{y}-C_{I}H_{2I}- (VIII)
mit k und l gleich unabhängig voneinander je 0 bis 8, x = 6 und y = 4 oder x = 10 und y = 6 oder x = 14 und y = 8, und
Z = O, CO, NH, NR¹, N-CH₂-CH(OX)-R¹, N-C(O)R¹, SO₂
mit R gleich unabhängig voneinander H oder C₁ - bis C₆-Alkyl
oder gemäß der Formel IX
-CH₂-CH(OCH₂CH(OX)-R¹)-CH₂- oder ein Isomer (IX)
mit X SO₃M,
wobei R¹ für einen Kohlenwasserstoffrest mit 1 bis 22 Kohlenstoffatomen steht,
◆ eine Gruppe gemäß der Formel X
-CₘH₂ₘ-(CₙH₂ₙO)ₚ(C₃H₆O)_{q}-CᵣH₂ᵣ- (X)
mit m = 1 bis 4, n = 2 bis 4, p = 1 bis 20, q = 0 bis 4 und r = 1 bis 4,
wobei auch gemischte Alkoxideinheiten auftreten können und dann die Reihenfolge der Alkoxideinheiten beliebig ist,
◆ 2,2'-Methylen-bis-(1,3-dioxolan-5-methylen)-
◆ Acetale,
◆ eine Gruppe gemäß der Formel XI
-CᵣH₂ᵣ(RNCₛH₂ₛ)ₜ-CᵤH₂ᵤ- (XI)
oder gemäß der Formel XII
-[CᵣH₂ᵣ[RN-C(O)-NR]ₜ-CᵤH₂ᵤ]_{w}- (XII)
oder gemäß der Formel XIII
-[CᵣH₂ᵣ[RNC(O)CᵥH₂ᵥC(O)NR]ₜ-CᵤH₂ᵤ]_{w}- (XIII)
oder gemäß der Formel XIV
-[CᵣH₂ᵣ[RNC(O)CₓR_{y}C(O)NR]ₜ-CᵤH₂ᵤ]_{w}- (XIV)
mit r = 2 bis 4, s = 2 bis 4, t = 1 bis 20, u = 2 bis 4, v = 0 bis 12, w = 1 bis 6,
x = 6 und y = 4 oder
x = 10 und y = 6 oder
x = 14 und y = 8
mit R gleich unabhängig voneinander H oder C₁- bis C₆-Alkyl,
und M und M' Alkali, Ammonium-, Alkanolammonium oder ½ Erdalkali bedeutet.

2. Amphiphile Verbindungen nach Anspruch 1,
dadurch gekennzeichnet,
daß die Alkylenkette der Formel II 2 bis 6 Kohlenstoffatome enthält.

3. Amphiphile Verbindungen nach Anspruch 1,
dadurch gekennzeichnet,
daß die Kohlenwasserstoffreste R¹ und R³ in der Formel I unabhängig voreinander 8 bis 18 Kohlenstoffatome enthalten.

4. Verwendung der amphiphilen Verbindungen nach mindestens einem der Ansprüche 1 bis 3 als Emulgatoren oder Demulgatoren.

5. Verwendung der amphiphilen Verbindungen nach mindestens einem der Ansprüche 1 bis 3 als Hilfsmittel bei der Metallbearbeitung, Erzgewinnung oder Oberflächenveredelung.

6. Verwendung der amphiphilen Verbindungen nach mindestens einem der Ansprüche 1 bis 3 als Textilhilfsmittel oder für das Reinigen und Waschen von Textilien.

7. Verwendung der amphiphilen Verbindungen nach mindestens einem der Ansprüche 1 bis 3 für das Reinigen von harten Oberflächen.

8. Verwendung der amphiphilen Verbindungen nach mindestens einem der Ansprüche 1 bis 3 für das Reinigen und Waschen von Haut und Haar.

## Claims

1. Amphiphilic compounds having the general formula I 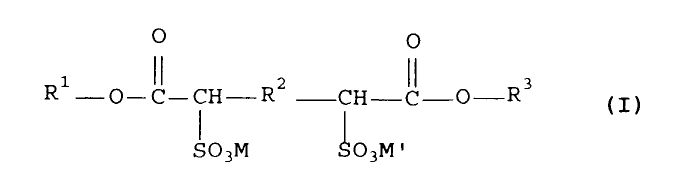
where **R**¹ and **R**³, independently of each other, are an unbranched or branched, saturated or unsaturated hydrocarbon radical having 1 to 22 carbon atoms,
**R**² is
◆ an unbranched or branched alkylene chain of formula II
-CₐH₂ₐ- (**II**)
where **a** = 2 to 18;
◆ an unbranched or branched alkynylene chain of formula III
-C_{d}H_{2d}-C≡C-CₑH₂ₑ- (**III**)
where **d** + **e** = 2 to 16, each **d** and **e** being greater than zero;
◆ alicyclic compounds of formula IV
-C_{f}H_{2f}-cycloC₆H₁₀-C_{g}H_{2g}- (**IV**)
where each **f** and **g**, independently of one another, is equal to from 1 to 6
or of formula V
-3(4),8(9)-di(methylene)-tricyclo[5.2.1.0^{2.6}] decane- (**V**)
◆ unsubstituted or substituted aromatics of formula VI
-CₕH₂ₕ-C₆R₄-(CᵢH₂ᵢ-C₆R₄)_{j₁}-C_{j₂}H_{2j₂}- (**VI**)
or of formula VII
-CₕH₂ₕ-C₁₀R₆-CⱼH₂ⱼ- (**VII**)
where each **h, j, j**₁, and **j**₂, independently of one another, is equal to from 0 to 8 and i is equal to from 0 to 8
and
each **R**, independently of one another, is equal to H or C₁- to C₆-alkyl;
◆ a group of formula VIII
-CₖH₂ₖ-CₓR_{y}-Z-CₓR_{y}-CₗH₂ₗ- (**VIII**)
where each **k** and **l**, independently of one another, is equal to from 0 to 8, **x** = 6 and **y** = 4, or **x** = 10 and **y** = 6, or **x** = 14 and **y** = 8, and Z = 0, CO, NH, NR¹, N-CH₂-CH(OX)-R¹, N-C(O)R¹,SO₂
wherein **R**, independently of one another, is equal to H or C₁- to C₆-alkyl
or of formula IX
-CH₂-CH(OCH₂CH(OX)-R¹)-CH₂- or an isomer (**IX**)
where **X** = SO₃M,
wherein **R**¹ is a hydrocarbon radical having 1 to 22 carbon atoms;
◆ a group of formula X
-CₘH₂ₘ-(CₙH₂ₙO)ₚ(C₃H₆O)_{q}-CᵣH₂ᵣ- (**X**)
where **m** = 1 to 4, **n** = 2 to 4, **p** = 1 to 20, **q** = 0 to 4, and **r** = 1 to 4, wherein also mixed alkoxide units may be present, and if so, the sequence of said alkoxide units is optional;
◆ 2,2'-methylene-bis-(1,3-dioxolane-5-methylene)-
◆ acetals
◆ a group of formula XI
-CᵣH₂ᵣ(RNCₛH₂ₛ)ₜ-CᵤH₂ᵤ- (**XI**)
or of formula XII
- [CᵣH₂ᵣ[RN-C(O)-NR]ₜ-CᵤH₂ᵤ]_{w}- (**XII**)
or of formula XIII
- [CᵣH₂ᵣ[RNC(O)CᵥH₂ᵥC(O)NR]ₜ-CᵤH₂ᵤ]_{w}- (**XIII**)
or of formula XIV
- [CᵣH₂ᵣ[RNC(O)CₓR_{y}C(O)NR]ₜ-CᵤH₂ᵤ]_{w}- (**XIV**)
where
**r** = 2 to 4, **s** = 2 to 4, **t** = 1 to 20,
**u** = 2 to 4, **v** = 0 to 12, **w** = 1 to 6,
**x** = 6 and **y** = 4, or
**x** = 10 and **y** = 6, or
**x** = 14 and y = 8,
wherein **R**, independently of one another, is equal to H or C₁- to C₆-alkyl,
and **M** and **M**' represent alkali, ammonium, alkanol ammonium, or ½ alkaline earth ion.

2. The amphiphilic compounds of claim 1,
**characterized in that**
the alkylene group of formula II has 2 to 6 carbon atoms.

3. The amphiphilic compounds of claim 1,
**characterized in that**
the hydrocarbon radicals **R**¹ and **R**³ in formula I, independently of one another, contain 8 to 18 carbon atoms.

4. Use of the amphiphilic compounds of at least one of claims 1 to 3 as emulsifiers or demulsifiers.

5. Use of the amphiphilic compounds of at least one of claims 1 to 3 as auxiliaries in metal working, ore mining, or surface finishing.

6. Use of the amphiphilic compounds of at least one of claims 1 to 3 as textile auxiliaries or for cleaning and washing textiles.

7. Use of the amphiphilic compounds of at least one of claims 1 to 3 for cleaning hard surfaces.

8. Use of the amphiphilic compounds of at least one of claims 1 to 3 for cleaning and washing skin and hair.

## Revendications

1. Composés amphiphiles de formule générale I : 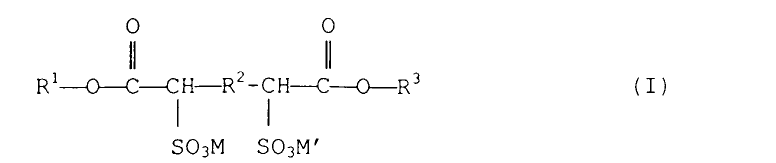
dans laquelle R¹ et R³ signifient, indépendamment l'un de l'autre, un radical hydrocarboné non ramifié ou ramifié, saturé ou insaturé, ayant 1 à 22 atomes de carbone,
R² signifie :
• une chaîne alkylène non ramifiée ou ramifiée de formule II :
-CₐH₂ₐ- (II)
avec a = 2 à 18,
• une chaîne alcinylène non ramifiée ou ramifiée de formule III :
-C_{d}H_{2d}-C≡C-CₑH₂ₑ- (III)
avec d + e = 2 à 16, d et e étant respectivement plus grands que zéro,
• un alicyclène de formule IV :
-C_{f}H_{2f}-cycloC₆H₁₀-C_{g}H_{2g}- (IV)
avec f et g égaux, indépendamment l'un de l'autre, respectivement à 1 à 6,
ou de formule V :
-3(4),8(9)-di(méthylène)-tricyclo[5.2.1.0^{2,6}]décan- (V)
• des aromatiques non substitués ou substitués de formule VI :
-CₕH₂ₕ-C₆R₄-(CᵢH₂ᵢ-C₆R₄)ⱼ₁-Cⱼ₂H₂ⱼ₂- (VI)
ou de formule VII :
-CₕH₂ₕ-C₁₀R₆-CⱼH₂ⱼ- (VII)
où h, j, j₁ et j₂ sont égaux, indépendamment les uns des autres, respectivement à 0 à 8 et 1= 0 à 8, et
R désignent, indépendamment les uns des autres, respectivement H ou alkyle en C₁-C₆,
• un groupe de formule VIII :
-CₖH₂ₖ-CₓR_{y}-Z-CₓR_{y}-CₗH₂ₗ- (VIII)
où k et l sont égaux, indépendamment l'un de l'autre, respectivement à 0 à 8, x = 6 et y = 4 ou x = 10 et y = 6 ou x = 14 et y = 8, et
Z = O, CO, NH, NR¹, N-CH₂-CH(OX)-R¹, N-C(O)R¹, SO₂,
R désignant, indépendamment les uns des autres, H ou un alkyle en C₁-C₆,
ou de formule IX :
-CH₂-CH(OCH₂CH(OX)-R¹)-CH₂ ou un isomère (IX)
avec X = SO₃M,
R¹ étant un radical hydrocarboné ayant 1 à 22 atomes de carbone,
• un groupe de formule X :
-CₘH₂ₘ-(CₙH₂ₙO)ₚ(C₃H₆O)_{q}-CᵣH₂ᵣ- (X)
avec m = 1 à 4, n = 2 à 4, p = 1 à 20, q = 0 à 4 et r = 1 à 4,
dans lequel il peut apparaître également des unités alcoxyde mixtes dont la séquence est alors quelconque,
• un 2,2'-méthylène-bis-(1,3-dioxolan-5-méthylène)-,
• des acétals,
• un groupe de formule XI :
-CᵣH₂ᵣ(RNCₛH₂ₛ)ₜ-CᵤH₂ᵤ- (XI)
ou de formule XII :
-[CᵣH₂ᵣ[RN-C(O)-NR]ₜ-CᵤH₂ᵤ]_{w}- (XII)
ou de formule XIII :
-[CᵣH₂ᵣ[RNC(O)CᵥH₂ᵥC(O)NR]ₜ-CᵤH₂ᵤ]_{w} (XIII)
ou de formule XIV :
-[CᵣH₂ᵣ[RNC(O)CₓR_{y}C(O)NR]ₜ-CᵤH₂ᵤ]_{w}- (XIV)
avec r = 2 à 4, s = 2 à 4, t = 1 à 20, u = 2 à 4,
v = 0 à 12, w = 1 à 6,
x = 6 et y = 4, ou
x = 10 et y = 6, ou
x = 14 et y = 8
R signifiant, indépendamment les uns des autres, H ou un alkyle en C₁-C₆,
et M et M' signifie alcali, ammonium, alcanolammonium ou ½ alcalinoterreux.

2. Composés amphiphiles selon la revendication 1, caractérisés en ce que la chaîne alkylène de formule II contient 2 à 6 atomes de carbone.

3. Composés amphiphiles selon la revendication 1, caractérisés en ce que les radicaux hydrocarbonés R¹ et R³ de la formule I contiennent, indépendamment l'un de l'autre, 8 à 18 atomes de carbone.

4. Utilisation des composés amphiphiles selon au moins l'une quelconque des revendications 1 à 3 comme émulsionnants ou désémulsionnants.

5. Utilisation des composés amphiphiles selon au moins l'une quelconque des revendications 1 à 3 comme auxiliaires dans le traitement des métaux, l'extraction de minerais ou l'ennoblissement de surfaces.

6. Utilisation des composés amphiphiles selon au moins l'une quelconque des revendications 1 à 3 comme auxiliaires textiles ou pour le nettoyage et le lavage de textiles.

7. Utilisation des composés amphiphiles selon au moins l'une quelconque des revendications 1 à 3 pour le nettoyage de surfaces dures.

8. Utilisation des composés amphiphiles selon au moins l'une quelconque des revendications 1 à 3 pour le nettoyage et le lavage des cheveux et de la peau.
